# EUROPÄISCHE PATENTSCHRIFT

(11) **EP 2 598 025 B1**
(45) Veröffentlichungstag und Bekanntmachung des Hinweises auf die Patenterteilung: **27.03.2019**
(21) Anmeldenummer: 11723915.2
(22) Anmeldetag: 25.05.2011
(51) Int. Cl.: A61B 5/083, A61B 10/00, A61B 5/09

(54) **VORRICHTUNG UND VERFAHREN ZUR BESTIMMUNG EINER FERTILEN PHASE EINER FRAU DURCH ERMITTLUNG EINES CO2-PARTIALDRUCKES IN EINEM ATEMGAS DER FRAU**
DEVICE AND METHOD FOR DETERMINING A FERTILE PHASE OF A WOMAN BY ASCERTAINING A CO2 PARTIAL PRESSURE IN A RESPIRATORY GAS OF THE WOMAN
DISPOSITIF ET PROCÉDÉ DE DÉTERMINATION D'UNE PHASE FERTILE CHEZ UNE FEMME EN DÉTECTANT UNE PRESSION PARTIELLE DE CO2 DANS UN GAZ RESPIRATOIRE DE LA LADITE FEMME

(30) Priorität: 25.05.2010 AT 8472010
(43) Veröffentlichungstag der Anmeldung: 05.06.2013
(73) Patentinhaber: Carbomed Medical Solutions GmbH, 8010 Graz (AT)
(72) Erfinder: Krammer, Gert, 8042 Graz (AT)
(74) Vertreter: Wirnsberger, Gernot
(86) Internationale Anmeldenummer: PCT/EP2011/058589
(87) Internationale Veröffentlichungsnummer: WO 2011/147888

(56) Entgegenhaltungen:
- WO-A2-00/28881
- WO-A2-98/49536
- DE-U1- 29 707 771
- US-A- 4 346 584
- US-A1- 2008 119 752

## Beschreibung

Die Erfindung betrifft eine Vorrichtung zur Bestimmung einer fertilen Phase einer Frau durch Ermittlung eines CO₂-Partialdruckes in einem Atemgas der Frau in mehreren aufeinanderfolgenden Atemzügen, umfassend einen Einlass zur Aufnahme des Atemgases und einen Auslass, eine Probenkammer, in welche das Atemgas leitbar ist, ein Messmodul mit einem Messelement, mit dem eine CO₂-Konzentration im Atemgas in der Probenkammer messbar ist, optional einen Drucksensor zur Messung eines Luftdruckes, eine Recheneinheit zur Verarbeitung von mit dem Messmodul und vom Drucksensor erhaltenen Messdaten und zumindest eine Ausgabeeinheit zur Ausgabe eines Ergebnisses der Messdaten.

Des Weiteren betrifft die Erfindung ein Verfahren zur Bestimmung einer fertilen Phase einer Frau durch Ermittlung eines endexspiratorischen CO₂-Partialdruckes in einem Atemgas der Frau und Vergleich der so ermittelten Messdaten mit Messdaten in der Follikelphase außerhalb der fertilen Phase.

Aus dem Stand der Technik ist es bekannt, dass sich eine Zusammensetzung eines Atemgases einer Frau im Menstruationszyklus verändert. Bereits etwa um das Jahr 1940 konnte gezeigt werden, dass ein endexspiratorischer CO₂-Partialdruck in der Lutealphase deutlich unter dem in der Follikelphase liegt. In einer detaillierten Studie wurde festgestellt, dass durch eine Messung des endexspiratorischen CO₂-Partialdruckes eine zuverlässige Zyklusüberwachung gewährleistet werden kann, da der endexspiratorische CO₂-Partialdruck bereits präovulatorisch am Beginn der fertilen Phase einsetzt (K.-T. Moeller et al., J. Fertil. Reprod. 2003, 7). Die in dieser Studie verwendeten Messapparaturen sind jedoch äußerst aufwendig und lassen kaum eine rasche Messung durch ungeschultes Personal, insbesondere eine Frau selbst, zu.

In der WO 98/49536 A2 ist eine Vorrichtung zur Bestimmung eines Ovulationszeitpunktes einer Frau offenbart, bei der ebenfalls eine Messung des CO₂-Partialdruckes im Atemgas einer Frau zu einer Aussage über die fertile Phase herangezogen wird. Die Vorrichtung kann klein und transportabel ausgebildet sein und ermöglicht eine Mitführung durch eine Benutzerin.

Im Rahmen der vorliegenden Erfindung wurde erkannt, dass für eine zuverlässige Messung des endexspiratorischen CO₂-Partialdruckes eine körperliche Ruhe der Frau bzw. der Testperson ausschlaggebend sein kann. Eine körperliche Ruhe äußert sich, wie erkannt wurde, in zumindest innerhalb gewisser Toleranzbereiche konstanten Messdaten bei unmittelbar aufeinanderfolgenden Atemzügen. Ist eine körperliche Ruhe gegeben und sind die Messdaten innerhalb der vorgegebenen Toleranzbereiche konstant, kann aber auch eine Messung rasch und genau abgeschlossen werden.

Auf Basis dieser Erkenntnis ist es Aufgabe der Erfindung, eine Vorrichtung der eingangs genannten Art anzugeben, mit der schnell und trotzdem genau eine Bestimmung der fertilen Phase auch durch nicht fachmännisches Personal oder die Frau selbst erfolgen kann.

Ein weiteres Ziel der Erfindung ist es, ein Verfahren der eingangs genannten Art anzugeben, mit dem durch eine Frau oder gegebenenfalls einen Dritten ohne fachmännische Assistenz auf schnelle und zuverlässige Weise eine fertile Phase bestimmt werden kann, insbesondere durch die Frau selbst.

Die Aufgabe wird erfindungsgemäß durch eine Vorrichtung gemäß Anspruch 1 und ein Verfahren gemäß Anspruch 7 gelöst.

Ein mit einer erfindungsgemäßen Vorrichtung erzielter Vorteil ist insbesondere darin zu sehen, dass mit einer kleinen, handgeführten bzw. handführbaren Vorrichtung schnell eine genaue Analyse erfolgen kann, ob eine fertile Phase einer Frau vorliegt. Dabei ist überdies von Vorteil, dass aufgrund des vorgesehenen Reproduzierbarkeitskriteriums eine Messzeit minimiert ist, weil z. B. ein Signal ausgegeben wird, sobald das Reproduzierbarkeitskriterium erfüllt ist. Sichergestellt ist auch, dass Fehlanalysen aufgrund körperlicher Belastung nahezu ausgeschlossen sind, da das Reproduzierbarkeitskriterium erst erfüllt ist, wenn dieses bei mehreren aufeinanderfolgenden Messungen erfüllt wird. Ist eine Frau außer Atem, beispielsweise infolge körperlicher Belastung, so wird die Messung so lange fortgeführt, bis eben das genannte Reproduzierbarkeitskriterium erreicht ist. Dies ermöglicht es, dass die Messung von ungeschultem, nicht fachmännischem Personal oder der Frau selbst durchgeführt werden kann, wobei in diesem Zusammenhang die Ausbildung der Vorrichtung als Handgerät zusätzlich von Vorteil ist, weil das Handgerät eine entsprechende Handlichkeit aufweist und die Frau beispielsweise bei der Messung liegen kann, um eine für die Messung erforderliche körperliche Ruhe herzustellen.

Anhand des Reproduzierbarkeitskriteriums werden einzelne Messungen des endexspiratorischen CO₂-Partialdruckes zueinander geprüft und bei Erreichen einer Reproduzierbarkeit für eine vorgegebene Anzahl von aufeinanderfolgenden Messungen die Messung abgeschlossen. Diesbezüglich wird vorab ein Toleranzfenster definiert, in dem die einzelnen Messdaten liegen müssen, um das Reproduzierbarkeitskriterium zu erfüllen.

Der Algorithmus errechnet anhand der Messdaten eines Zyklus eine Grundlinie für den endexspiratorischen CO₂-Partialdruck in der Follikelphase außerhalb der fertilen Phase, damit in der Folge Abweichungen von der Grundlinie und damit eine fertile Phase zuverlässig erkennbar ist.

Des Weiteren kann bevorzugt vorgesehen sein, dass der Algorithmus Messdaten des endexspiratorischen CO₂-Partialdruckes mehrerer vorangegangener Zyklen bei einer Interpretation der Messdaten berücksichtigt.

Sofern mehrere Messungen an einem Tag durchgeführt werden, was in Anbetracht eines Herannahens einer fertilen Phase durchaus vorkommen kann, berechnet der Algorithmus ein gleitendes Tagesmittel, sodass auch in dieser Hinsicht eine Mittelung und damit eine Verfeinerung der Messdaten erfolgt bzw. eine höhere Aussagekraft des Ergebnisses derselben gegeben ist.

Die Ausgabeeinheit kann bei Erreichen des Reproduzierbarkeitskriteriums eine grundsätzlich beliebige Ausgabe vornehmen. Möglich ist es beispielsweise, dass ein Bon ausgegeben wird, auf welchem die Messdaten samt Ergebnis vermerkt sind. Möglich ist es ebenfalls, dass die Messdaten bzw. ein Ergebnis der Messung unmittelbar auf der Vorrichtung mittels farblicher Codierung bzw. Farbcode auf einem Display der Vorrichtung dargestellt wird. Von Vorteil kann es alternativ oder gleichzeitig auch sein, da die Vorrichtung handgeführt ist und während der Messung eine Sicht auf die Vorrichtung verstellt sein kann, insbesondere wenn die Frau selbst misst und dabei liegt, dass ein akustisches Signal ausgebbar ist.

Besonders bevorzugt ist es auch, dass ein Temperatursensor in der Probenkammer vorgesehen ist. Alternativ können auch ein Temperatursensor und ein Feuchtigkeitssensor in der Probenkammer vorgesehen sein. Dadurch kann eine Korrektur in Bezug auf einen Wasserdampfpartialdruck erfolgen, der unter Umständen ein Ergebnis um bis zu 5 % verfälschen kann.

Das Messmodul und das zugehörige Messelement können an sich beliebig ausgebildet sein. Bevorzugt umfasst das Messmodul jedoch ein Messelement, das mit IR-Strahlung einer oder mehrerer Wellenlängen arbeitet. Beispielsweise kann ein IR-Spektroskop vorgesehen sein. Mit einem IR-Spektroskop kann auf besonders einfache Weise eine CO₂-Konzentration in einem Atemgas bestimmt werden. Das IR-Spektroskop kann gegebenenfalls auch ein photoakustisches IR-Spektroskop sein.

Alternativ kann das Messmodul einen elektrochemischen Sensor oder einen Schallwellensensor zur Messung der CO₂-Konzentration umfassen.

Insbesondere für eine Messung in einem Hauptstromverfahren ist es günstig, wenn das Messmodul einen Festkörpersensor zur Messung der CO₂-Konzentration auf Basis einer lonenleitfähigkeit umfasst. Ein derartiges Verfahren erfüllt die Anforderungen an eine schnelle Messung im Hauptstromverfahren.

Möglich ist es auch, dass mehrere Sensoren gleichzeitig vorgesehen sind, wobei die Ergebnisse aller Sensoren berücksichtigt werden können.

Im Hinblick auf möglichst präzise Messdaten und eine Vermeidung einer Kondensatbildung kann es auch bevorzugt sein, dass eine Einrichtung zur Thermostatisierung der Probenkammer vorgesehen ist.

Eine Messung kann im Hauptstromverfahren oder im Nebenstromverfahren durchgeführt werden. Das Hauptstromverfahren ist grundsätzlich bevorzugt, da dem Atemgas nur ein sehr geringer Strömungswiderstand entgegengesetzt wird. Möglich ist es aber auch, nur im ausgeatmeten Atemgas eine Messung durchzuführen, wobei hierfür vorteilhafterweise eine Pumpe vorgesehen ist, mit der ein bestimmtes Volumen des Atemgases aus einem zwischen dem Einlass und der Probenkammer liegenden Raum in die Probenkammer leitbar ist.

Die Vorrichtung kann des Weiteren ein Mundstück umfassen. Grundsätzlich ist es möglich, dass Atemgas bzw. ein Probenvolumen durch eine Atemmaske oder eventuell auch eine Nasensonde zur Messung in der Probenkammer bereitgestellt wird. Ein Mundstück ist jedoch die einfachste Alternative und hat sich auch in Bezug auf eine Genauigkeit als durchaus praktikabel erwiesen.

In der Probenkammer kann ein weiteres Messelement, z. B. ein beheiztes Widerstandselement oder ein Propeller, vorgesehen sein, mit dem eine Atemgeschwindigkeit messbar ist. Durch eine Messung der Atemgeschwindigkeit kann eine zusätzliche Variable beim Reproduzierbarkeitskriterium berücksichtigt werden.

Das weitere Ziel der Erfindung wird durch ein Verfahren gemäß Anspruch 7 erreicht. Ein mit einem erfindungsgemäßen Verfahren erzielter Vorteil ist darin zu sehen, dass das Verfahren eine schnelle und trotzdem genaue und zuverlässige Analyse hinsichtlich einer möglichen fertilen Phase einer Frau erlaubt. Das Verfahren kann gegebenenfalls auch von der Frau selbst ausgeführt werden, wobei bei Erreichen des Reproduzierbarkeitskriteriums die Messung beendet und von der Ausgabeeinheit beispielsweise ein Signal ausgegeben wird, sodass die Frau das Ende der Messung passiv erkennt. Daneben wird durch die Ausgabeeinheit das Ergebnis der Messung ausgegeben, signalisiert und/oder auf andere Weise dargestellt. Diesbezüglich ist es möglich, dass zuerst ein Ende der Messung z. B. akustisch signalisiert und anschließend auf einem Display ein Messergebnis dargestellt wird. Es kann aber auch sogleich mit dem Ende der Messung das Messergebnis ausgegeben werden, beispielsweise durch Worte (fruchtbar bzw. nicht fruchtbar) über einen kleinen Lautsprecher.

Anhand der Messdaten eines Zyklus wird eine Grundlinie für den CO₂-Partialdruck in der Follikelphase außerhalb der fertilen Phase errechnet, damit in der Folge Abweichungen von der Grundlinie und damit eine fertile Phase zuverlässig erkennbar ist.

Besonders bevorzugt ist es, dass die Messdaten mehrerer vorangegangener Zyklen bei einer Interpretation der Messdaten berücksichtigt werden. Dadurch kann eine Messgenauigkeit und somit auch eine Genauigkeit der Vorhersage fruchtbarer Tage erhöht werden.

Bei einer Mehrfachmessung an einem Tag wird im Hinblick auf eine hohe Messgenauigkeit bevorzugt ein gleitendes Tagesmittel berechnet.

Ein Ende der Messung bzw. ein Erreichen des Reproduzierbarkeitskriteriums kann beispielsweise durch Anzeige auf einem Display der verwendeten Vorrichtung oder Ausgabe eines Messungsbelegs erfolgen. Bevorzugt ist jedoch vorgesehen, insbesondere wenn die eingesetzte Vorrichtung ein Handgerät ist, dass bei Erreichen des Reproduzierbarkeitskriteriums ein akustisches Signal ausgegeben wird. Die Frau kann sich dann bei der Messung vollständig auf eine ruhige Atmung konzentrieren, ohne auf die Vorrichtung sehen zu müssen. Dadurch ergibt sich auch der Vorteil, dass die Vorrichtung besonders klein ausgebildet werden kann, insbesondere mit einer Größe, die etwa der Größe einer Hand entspricht. Alternativ kann auch eine farbcodierte Darstellung erfolgen, wenn eine Sicht auf ein Display der Vorrichtung nicht eingeschränkt ist.

Die Ermittlung des CO₂-Partialdruckes kann auf beliebige Art erfolgen. Bevorzugt ist vorgesehen, dass die Ermittlung der CO₂-Konzentration durch ein IR-Messverfahren durchgeführt wird, da sich durch diese Methode zuverlässige Messergebnisse erreichen lassen.

Mit Vorteil ist vorgesehen, dass ein Wasserdampfpartialdruck aus einer Temperaturmessung in der Probenkammer und/oder einer Temperaturmessung und einer Messung einer Luftfeuchtigkeit in der Probenkammer berechnet und als Korrekturgröße berücksichtigt wird. Versuche haben gezeigt, dass die Messergebnisse um bis zu 5 % in ihrer Genauigkeit verbessert werden können, wenn die Messdaten entsprechend korrigiert werden.

Zur Verbesserung einer Aussagekraft der Messung bzw. Minimierung eines Risikos kann ein Messergebnis mit einem Messergebnis einer weiteren Methode zur Bestimmung einer fertilen Phase der Frau, vorzugsweise einer Basaltemperaturbestimmung, kombiniert werden.

Es kann vorgesehen sein, dass die Messdaten mehrerer vorangegangener Zyklen gespeichert werden und abrufbar sind, sodass für eine Benutzerin ein Vergleich mit älteren Messergebnissen möglich ist.

Um ein genaues Messergebnis zu erreichen, kann vor einer ersten Messung und/oder zwischen Messungen eine Kalibration mittels bekannter Gaskonzentrationen durchgeführt werden.

In der Probenkammer kann mit einem weiteren Messelement, z. B. einem beheizten Widerstandselement oder einem Propeller, eine Atemgeschwindigkeit gemessen und vorzugsweise als Variable bei der Erfüllung des Reproduzierbarkeitskriteriums berücksichtigt werden.

Weitere Merkmale, Vorteile und Wirkungen der Erfindung ergeben sich aus dem nachfolgend dargestellten Ausführungsbeispiel. In der Zeichnung, auf welche dabei Bezug genommen wird, zeigen:
Fig. 1 eine schematische Darstellung eines Messaufbaus, der für ein Haupt- oder Nebenstromverfahren genutzt werden kann;
Fig. 2 eine schematische Darstellung eines Nebenstromverfahrens.

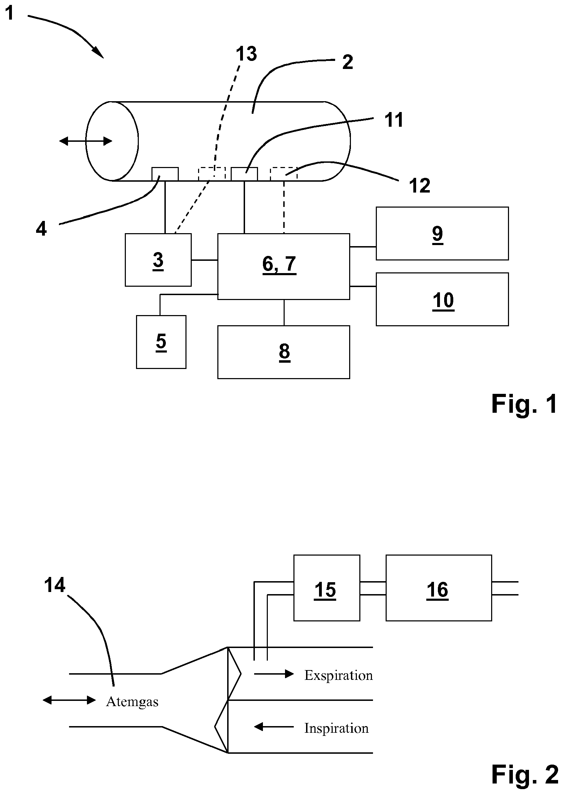

In Fig. 1 ist eine Vorrichtung 1 bzw. ein Messaufbau schematisch dargestellt. Die Vorrichtung 1 ist als Handgerät ausgebildet, insbesondere mit einem Kunststoffgehäuse, das von einer Frau einfach mit einer Hand gehalten werden kann. Darüber hinaus weist die bevorzugt länglich ausgebildete Vorrichtung 1 ein endseitiges Mundstück auf, in welches von der Frau geatmet werden kann.

Die Vorrichtung 1 umfasst eine rohrförmige Probenkammer 2 mit einem Messelement 4 zur Bestimmung einer CO₂-Konzentration in einem Atemgas. Des Weiteren ist ein Messmodul 3 vorgesehen, das zum Betrieb des Messelementes 4 und zur Amplifizierung eines Messsignals eingesetzt werden kann und zu diesen Zwecken mit dem Messelement 4 in Verbindung steht. Das Messelement 4 arbeitet bevorzugt mit IR-Strahlung einer oder mehrerer Wellenlängen, sodass durch Messung im infraroten Bereich des elektromagnetischen Spektrums eine CO₂-Konzentration in der Probenkammer 2 von dem dort befindlichen Atemgas ermittelt werden kann. Das Messmodul 3 steht mit einem Analog-Digital-Konverter 6 bzw. einer Recheneinheit 7 in Verbindung. Die Recheneinheit 7 wiederum steht mit einem Speicher 8 und einem Drucksensor 5 für die Erfassung eines Luftdruckes in Verbindung; der Drucksensor 5 ist nicht zwingend, wenn die Vorrichtung 1 vor erstmaligem Gebrauch mit einem externen Sensor kalibriert wird. Des Weiteren sind eine Eingabeeinheit 10 sowie eine Ausgabeeinheit 9 vorgesehen. In der Probenkammer 2 befinden sich zusätzlich ein Temperatursensor 11 sowie mit Vorteil auch ein Feuchtigkeitssensor 12. Durch diese zusätzlichen Sensoren ist es möglich, Messdaten hinsichtlich eines Wasserdampfpartialdruckes des Atemgases zu korrigieren. Des Weiteren kann ein beheiztes Widerstandselement 13 zur Messung einer Atemgeschwindigkeit in der Probenkammer 2 vorgesehen sein, damit die Atemgeschwindigkeit als Kontrollgröße bei der Auswertung der Messdaten berücksichtigt werden kann.

In Fig. 2 ist ein Nebenstromverfahren dargestellt, bei dem das Atemgas über ein Ventilsystem gelenkt wird. Vorteilhafterweise wird ein vorbestimmtes, konstantes Volumen des ausgeatmeten Gases aus einem Einlassbereich 14 mithilfe einer Pumpe 15 zu einem Messaufbau 16 gemäß Fig. 1 transportiert.

Bei der Durchführung einer Messung, die von einer Frau selbst durchgeführt werden kann, nimmt die Frau die Vorrichtung 1 und setzt das Mundstück an, um danach bei körperlicher Ruhe ein- und auszuatmen. Mit dem Messmodul 3 wird dabei eine endexspiratorische CO₂-Konzentration gemessen. Danach werden die Messdaten mithilfe des ebenfalls gemessenen aktuellen Luftdruckes in einen Partialdruck umgewandelt und um den Wasserdampfpartialdruck korrigiert. Die nacheinander aufgenommenen Messdaten werden gespeichert und hinsichtlich eines Reproduzierbarkeitskriteriums geprüft. Das Reproduzierbarkeitskriterium wird im Vorhinein festgelegt. Beispielsweise kann vorgesehen sein, dass das Reproduzierbarkeitskriterium erfüllt ist, wenn mehrere, z. B. fünf, aufeinanderfolgende Messungen innerhalb eines vorgegebenen Signalfensters von beispielsweise 1 mm Hg des CO₂-Partialdruckes liegen. Es versteht sich, dass das Signalfenster grundsätzlich beliebig gesetzt werden kann. Zweckmäßigerweise wird das Signalfenster so gewählt, dass einerseits eine ausreichende Genauigkeit einer Messung sichergestellt ist, andererseits hierfür aber auch ein Minimum an Wiederholungen ausreichend ist. Sobald das Reproduzierbarkeitskriterium erfüllt ist, gibt die Ausgabeeinheit 9 der Vorrichtung 1 ein vorzugsweise akustisches Signal und/oder eine farbcodierte Darstellung, gegebenenfalls auf einem Display der Vorrichtung, aus, sodass die Benutzerin die Messung beenden kann; gleichzeitig erfolgt eine Darstellung des Messergebnisses, z. B. an einem Display der Vorrichtung 1. Durch diese Vorgehensweise ist sichergestellt, dass rasch zuverlässige Messdaten erhalten werden, auch wenn kein fachmännisches Personal anwesend ist.

## Patentansprüche

1. Vorrichtung (1) zur Bestimmung einer fertilen Phase einer Frau durch Ermittlung eines CO₂-Partialdruckes in einem Atemgas der Frau in mehreren aufeinanderfolgenden Atemzügen, umfassend einen Einlass zur Aufnahme des Atemgases und einen Auslass, eine Probenkammer (2), in welche das Atemgas leitbar ist, ein Messmodul (3) mit einem Messelement (4), mit dem eine CO₂-Konzentration im Atemgas in der Probenkammer (2) messbar ist, optional einen Drucksensor (5) zur Messung eines Luftdruckes, eine Recheneinheit (7) zur Verarbeitung von mit dem Messmodul (3) und vom Drucksensor (5) erhaltenen Messdaten und zumindest eine Ausgabeeinheit (9) zur Ausgabe eines Ergebnisses der Messdaten, wobei die Vorrichtung (1) als Handgerät ausgebildet ist, welches von einer Frau mit einer Hand haltbar ist, und in der Recheneinheit (7) ein Algorithmus gespeichert ist, mit dem bei einer Messung anhand mehrerer unmittelbar aufeinanderfolgender Atemzüge ein bezüglich eines endexspiratorischen CO₂-Partialdruckes des Atemgases vorgegebenes Reproduzierbarkeitskriterium geprüft wird, wobei bei Erfüllung des Reproduzierbarkeitskriteriums die zumindest eine Ausgabeeinheit (9) ausgibt und/oder signalisiert und/oder darstellt, ob eine fertile Phase vorliegt, **dadurch gekennzeichnet, dass** der Algorithmus anhand der Messdaten eines Zyklus eine Grundlinie für den endexspiratorischen CO₂-Partialdruck in der Follikelphase außerhalb der fertilen Phase errechnet und vorab ein Toleranzfenster definiert ist, in dem die einzelnen Messwerte liegen müssen, um das Reproduzierbarkeitskriterium zu erfüllen.

2. Vorrichtung (1) nach Anspruch 1, **dadurch gekennzeichnet, dass** der Algorithmus Messdaten des endexspiratorischen CO₂-Partialdruckes mehrerer vorangegangener Zyklen bei einer Interpretation der Messdaten berücksichtigt.

3. Vorrichtung (1) nach Anspruch 1 oder 2, **dadurch gekennzeichnet, dass** der Algorithmus bei Mehrfachmessung an einem Tag ein gleitendes Tagesmittel berechnet.

4. Vorrichtung (1) nach einem der Ansprüche 1 bis 3, **dadurch gekennzeichnet, dass** mit der Ausgabeeinheit (9) bei Erreichen des Reproduzierbarkeitskriteriums ein Farbcode und/oder ein akustisches Signal ausgebbar ist.

5. Vorrichtung (1) nach einem der Ansprüche 1 bis 4, **dadurch gekennzeichnet, dass** ein Temperatursensor (11) in der Probenkammer (2) vorgesehen ist.

6. Vorrichtung (1) nach Anspruch 5, **dadurch gekennzeichnet, dass** ein Feuchtigkeitssensor (12) in der Probenkammer (2) vorgesehen ist.

7. Verfahren zur Bestimmung einer fertilen Phase einer Frau durch Ermittlung eines endexspiratorischen CO₂-Partialdruckes in einem Atemgas der Frau und Vergleich der so ermittelten Messdaten mit Messdaten in der Follikelphase außerhalb der fertilen Phase, wobei bei einer Messung in einer Probenkammer (2), insbesondere mit einer Vorrichtung (1) gemäß einem der Ansprüche 1 bis 6, anhand mehrerer unmittelbar aufeinanderfolgender Atemzüge ein bezüglich des endexspiratorischen CO₂-Partialdruckes des Atemgases vorgegebenes Reproduzierbarkeitskriterium geprüft wird und zumindest eine Ausgabeeinheit (9) ein Signal ausgibt, wenn das Reproduzierbarkeitskriterium für eine vorbestimmte Anzahl von Atemzügen erreicht ist, und von der Ausgabeeinheit (9) ausgegeben und/oder signalisiert und/oder dargestellt wird, ob eine fertile Phase vorliegt, **dadurch gekennzeichnet, dass** anhand der Messdaten eines Zyklus eine Grundlinie für den endexspiratorischen CO₂-Partialdruck in der Follikelphase außerhalb der fertilen Phase errechnet wird und vorab ein Toleranzfenster definiert wird, in dem die einzelnen Messwerte liegen müssen, um das Reproduzierbarkeitskriterium zu erfüllen.

8. Verfahren nach Anspruch 7, **dadurch gekennzeichnet, dass** die Messdaten mehrerer vorangegangener Zyklen bei einer Interpretation der Messdaten berücksichtigt werden.

9. Verfahren nach Anspruch 7 oder 8, **dadurch gekennzeichnet, dass** bei Mehrfachmessung an einem Tag ein gleitendes Tagesmittel berechnet wird.

10. Verfahren nach einem der Ansprüche 7 bis 9, **dadurch gekennzeichnet, dass** bei Erreichen des Reproduzierbarkeitskriteriums ein akustisches Signal und/oder eine farbcodierte Darstellung ausgegeben wird.

11. Verfahren nach einem der Ansprüche 7 bis 10, **dadurch gekennzeichnet, dass** die Ermittlung der CO₂-Konzentration durch ein IR-Messverfahren durchgeführt wird.

12. Verfahren nach einem der Ansprüche 7 bis 11, **dadurch gekennzeichnet, dass** ein Wasserdampfpartialdruck aus einer Temperaturmessung in der Probenkammer (2) und/oder einer Temperaturmessung und einer Messung einer Luftfeuchtigkeit in der Probenkammer (2) berechnet und als Korrekturgröße berücksichtigt wird.

13. Verfahren nach einem der Ansprüche 7 bis 12, **dadurch gekennzeichnet, dass** ein Messergebnis mit einem Messergebnis einer weiteren Methode zur Bestimmung einer fertilen Phase der Frau, vorzugsweise einer Basaltemperaturbestimmung, kombiniert wird.

## Claims

1. A device (1) for determining a fertile phase of a woman by ascertaining a CO₂ partial pressure in a respiratory gas from the woman in a plurality of consecutive breaths, comprising an inlet for receiving the respiratory gas and an outlet, a sample chamber (2) into which the respiratory gas can be conducted, a measuring module (3) with a measuring element (4) with which a CO₂ concentration in the respiratory gas in the sample chamber (2) can be measured, optionally a pressure sensor (5) for measuring an air pressure, a processing unit (7) for processing measured data obtained with the measuring module (3) and from the pressure sensor (5), and at least one output unit (9) for outputting a result of the measured data, wherein the device (1) is configured as a hand-held device which can be held by a woman in one hand, and an algorithm stored in the processing unit (7) with which, when a measurement based on a plurality of immediately consecutive breaths is carried out, a predetermined reproducibility criterion relating to an end expiratory CO₂ partial pressure of the respiratory gas can be tested, wherein, when the reproducibility criterion is met, the at least one output unit (9) outputs and/or signals and/or displays whether a fertile phase is present, **characterized in that** based on the measured data of a cycle, the algorithm calculates a base line for the end expiratory CO₂ partial pressure in the follicle phase outside the fertile phase, and in advance, a tolerance range is defined in which the individual measured values must lie in order to meet the reproducibility criterion.

2. The device (1) as claimed in claim 1, **characterized in that** the algorithm takes into consideration measured data of the end expiratory CO₂ partial pressure of a plurality of preceding cycles when an interpretation of the measured data is carried out.

3. The device (1) as claimed in claim 1 or claim 2, **characterized in that** when a plurality of measurements is carried out on one day, the algorithm calculates a sliding daily average.

4. The device (1) as claimed in one of claims 1 to 3, **characterized in that** when the reproducibility criterion is met, a colour code and/or an acoustic signal can be output with the output device (9).

5. The device (1) as claimed in one of claims 1 to 4, **characterized in that** a temperature sensor (11) is provided in the sample chamber (2).

6. The device (1) as claimed in claim 5, **characterized in that** a moisture sensor (12) is provided in the sample chamber (2).

7. A method for determining a fertile phase of a woman by ascertaining an end expiratory CO₂ partial pressure in a respiratory gas from the woman and comparing the measured data thus obtained with measured data in the follicle phase outside the fertile phase wherein, when a measurement is carried out in a sample chamber (2), in particular with a device (1) as claimed in one of claims 1 to 6, based on a plurality of immediately consecutive breaths, a predetermined reproducibility criterion relating to the end expiratory CO₂ partial pressure of the respiratory gas is tested and at least one output unit (9) outputs a signal when the reproducibility criterion for a predetermined number of breaths has been met and the output unit (9) outputs and/or signals and/or displays whether a fertile phase is present, **characterized in that**, based on the measured data of a cycle, a base line for the end expiratory CO₂ partial pressure in the follicle phase outside the fertile phase is calculated, and in advance, a tolerance range is defined in which the individual measured values must lie in order to meet the reproducibility criterion.

8. The method as claimed in claim 7, **characterized in that** the measured data of a plurality of preceding cycles are taken into consideration when an interpretation of the measured data is carried out.

9. The method as claimed in claim 7 or claim 8, **characterized in that** when a plurality of measurements is carried out on one day, a sliding daily average is calculated.

10. The method as claimed in one of claims 7 to 9, **characterized in that** when the reproducibility criterion is met, an acoustic signal and/or a colour-coded display is output.

11. The method as claimed in one of claims 7 to 10, **characterized in that** the CO₂ concentration is determined by an IR measuring method.

12. The method as claimed in one of claims 7 to 11, **characterized in that** a water vapour partial pressure is calculated from a temperature measurement in the sample chamber (2) and/or from a temperature measurement and a measurement of an air humidity in the sample chamber (2) and is taken into consideration as a correction variable.

13. The method as claimed in one of claims 7 to 12, **characterized in that** a measured result is combined with a measured result from a further method for determining a fertile phase of the woman, preferably from a basal temperature determination.

## Revendications

1. Dispositif (1), destiné à déterminer une phase fertile chez la femme, par la recherche d'une pression partielle de CO₂ dans un gaz respiratoire de la femme, dans plusieurs souffles consécutifs, comprenant une entrée destinée à recevoir le gaz respiratoire et une sortie, une chambre d'échantillonnage (2) dans laquelle le gaz respiratoire peut être dirigé, un module de mesure (3) pourvu d'un élément de mesure (4), permettant de mesurer une concentration de CO₂ dans le gaz respiratoire dans la chambre d'échantillonnage (2), en option un capteur de pression (5), destiné à mesurer une pression atmosphérique, une unité informatique (7), destinée à traiter des données de mesure obtenues à l'aide du module de mesure (3) et à partir du capteur de pression (5) et au moins une unité d'édition (9), destinée à éditer un résultat des données de mesure, le dispositif (1) étant conçu sous la forme d'un appareil portable susceptible de tenir dans une seule main de la femme et dans l'unité informatique (7) étant mémorisé un algorithme à l'aide duquel lors d'une mesure sur la base de plusieurs souffles immédiatement successifs, un critère de reproductibilité prédéfini par rapport à une pression partielle de CO₂ en fin d'expiration finale est vérifié, lorsque le critère de reproductibilité est satisfait, l'au moins une unité d'édition (9) éditant et/ou signalant et/ou représentant si on est en présence d'une phase fertile, **caractérisé en ce qu'**à l'aide des données de mesure d'un cycle, l'algorithme calcule une ligne de fond pour la pression partielle de CO₂ en fin d'expiration dans la phase folliculaire, hors de la phase fertile et qu'en amont, il est défini une fenêtre de tolérance dans laquelle doivent se situer les valeurs de mesure individuelles, pour satisfaire au critère de reproductibilité.

2. Dispositif (1) selon la revendication 1, **caractérisé en ce que** lors de l'interprétation des données de mesure, l'algorithme tient compte de données de mesure de la pression partielle de CO₂ en fin d'expiration finale pour plusieurs cycles précédents.

3. Dispositif (1) selon la revendication 1 ou 2, **caractérisé en ce que** lors d'une mesure multiple sur une journée, l'algorithme calcule une moyenne journalière pondérée.

4. Dispositif (1) selon l'une quelconque des revendications 1 à 3, **caractérisé en ce qu'**à l'aide de l'unité d'édition (9), un code couleur et/ou un signal acoustique peut être édité à l'atteinte du critère de reproductibilité.

5. Dispositif (1) selon l'une quelconque des revendications 1 à 4, **caractérisé en ce qu'**un capteur de température (11) est prévu dans la chambre d'échantillonnage (2).

6. Dispositif (1) selon la revendication 5, **caractérisé en ce qu'**un capteur d'humidité (12) est prévu dans la chambre d'échantillonnage (2).

7. Procédé, destiné à destiné à déterminer une phase fertile chez la femme, par la recherche d'une pression partielle de CO₂ dans un gaz respiratoire de la femme, et à comparer les données de mesure ainsi recherchées avec des données de mesure dans la phase folliculaire hors de la phase fertile, lors d'une mesure dans une chambre d'échantillonnage (2), notamment à l'aide d'un dispositif (1) selon l'une quelconque des revendications 1 à 6, sur la base de plusieurs souffles immédiatement successifs, un critère de reproductibilité prédéfini par rapport à une pression partielle de CO₂ en fin d'expiration finale étant vérifié et au moins une unité d'édition (9) délivrant un signal lorsque le critère de reproductibilité est atteint pour un nombre prédéfini de souffles et il est édité et/ou signalé et/ou représenté par l'unité d'édition (9) si on est en présence d'une phase fertile, **caractérisé en ce qu'**à l'aide des données de mesure d'un cycle, une ligne de fond est calculée pour la pression partielle de CO₂ en fin d'expiration finale dans la phase folliculaire, hors de la phase fertile et une fenêtre de tolérance est définie dans laquelle doivent se situer les valeurs de mesure individuelles, pour satisfaire au critère de reproductibilité.

8. Procédé selon la revendication 7, **caractérisé en ce que** les données de mesure de plusieurs cycles précédents sont prises en compte lors de l'interprétation des données de mesure.

9. Procédé selon la revendication 7 ou 8, **caractérisé en ce que** lors d'une mesure multiple sur une journée, une moyenne journalière pondérée est calculée.

10. Procédé selon l'une quelconque des revendications 7 à 9, **caractérisé en ce qu'**à l'atteinte du critère de reproductibilité, un signal acoustique et/ou une représentation en code couleur est édité(e).

11. Procédé selon l'une quelconque des revendications 7 à 10, **caractérisé en ce que** la recherche de la concentration de CO₂ est réalisée à l'aide d'un procédé de mesure par IR.

12. Procédé selon l'une quelconque des revendications 7 à 11, **caractérisé en ce qu'**une pression partielle de vapeur d'eau est calculée à partir d'une mesure de la température dans la chambre d'échantillonnage (2) et/ou d'une mesure de la température et d'une mesure de l'humidité atmosphérique dans la chambre d'échantillonnage (2) et prise en compte comme grandeur de correction.

13. Procédé selon l'une quelconque des revendications 7 à 12, **caractérisé en ce qu'**un résultat de mesure est associé avec un résultat de mesure d'une autre méthode destinée à déterminer une phase fertile de la femme, de préférence de la méthode des températures.
